(19) Europäisches Patentamt
European Patent Office
Office européen des brevets

(11) Veröffentlichungsnummer: **0 250 648 B1**

(12) **EUROPÄISCHE PATENTSCHRIFT**

(45) Veröffentlichungstag der Patentschrift: **21.08.91**

(51) Int. Cl.5: **A61K 9/52**, A61K 9/20, A61K 31/19

(21) Anmeldenummer: **86117458.9**

(22) Anmeldetag: **16.12.86**

(54) **Pharmazeutisches Präparat zur verzögerten Freigabe von Ibuprofen.**

(30) Priorität: **25.06.86 CH 2553/86**

(43) Veröffentlichungstag der Anmeldung:
**07.01.88 Patentblatt 88/01**

(45) Bekanntmachung des Hinweises auf die Patenterteilung:
**21.08.91 Patentblatt 91/34**

(84) Benannte Vertragsstaaten:
**AT BE DE ES FR GB GR IT LU NL SE**

(56) Entgegenhaltungen:
**EP-A- 0 052 075**
**EP-A- 0 153 104**
**EP-A- 0 172 014**
**GB-A- 2 132 887**
**GB-A- 2 178 313**

(73) Patentinhaber: **Mepha AG**
**Dornacherstrasse 114**
**CH-4147 Aesch(CH)**

(72) Erfinder: **Seth, Pyare Lal, Dr.**
**Krebsenbachweg 8**
**CH-4147 Aesch(CH)**

(74) Vertreter: **Frossard, Michel, Dr. et al**
**A. Braun, Braun, Héritier, Eschmann AG, Patentanwälte Holbeinstrasse 36-38**
**CH-4051 Basel(CH)**

EP 0 250 648 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Der unter dem Namen Ibuprofen bekannte Wirkstoff 2-(4-Isobutylphenyl)-propionsäure findet wegen der guten analgetischen und antiphlogistischen Eigenschaften zur Behandlung von leichten bis mittelstarken Schmerzzuständen, bei Weichteilrheumatismus und bei entzündlichen und degenerativen Gelenkerkrankungen breite Verwendung. Gegenüber anderen, nichtsteroidalen antiphlogistischen Präparaten zeichnet sich Ibuprofen u.a. durch seine gute Magenverträglichkeit aus.

Zur Behandlung insbesondere der rheumatischen Arthritis und der Osteoarthritis wird eine Dosis von 900 bis 1'600 mg/Tag empfohlen (L.S. Goodman u. A. Gilman, The Pharmacological Basis of Therapeutics, 5. Auflage, MacMillan Publ. Co., New York 1975, Seite 343). Nach Arzneimittel-Profile/Basisinformation über arzneiliche Wirkstoffe [Govi-Verlag GmbH und Pharmazeutischer Verlag, Frankfurt a/Main (BRD), 2. Ergänzungslieferung, November 1983, Ibuprofen] liegt die Tagesdosis bei 1'200 bis 1'600 mg; sie kann in Einzelfällen auf 2'400 mg/Tag gesteigert, soll jedoch nicht darüber erhöht werden.

Eine derart hohe Dosierung kann deshalb ohne Bedenken angewendet werden, weil der Wirkstoff rasch und restlos aus dem Körper ausgeschieden wird: 24 Stunden nach der letzten Verabreichung ist mit einer vollständigen Elimination von Ibuprofen und seiner Metaboliten zu rechnen (Arzneimittel-Profile, ibidem).

Diese rasche Elimination hat allerdings eine Kehrseite. Um eine über den Tag wirksame Wirkstoffkonzentration im Organismus und namentlich im Plasma zu gewährleisten, muss die Verabreichung von Ibuprofen alle paar Stunden wiederholt werden. Das Dosierungsschema für Erwachsene ist z.B. 3 mal täglich 1 bis 2 Tabletten zu 200 mg, 3 oder 4 mal täglich 1 Tablette zu 400 mg, oder 400 mg alle 4 bis 6 Stunden.

Bei dieser häufigen Einnahme kann beim Patienten auf die Dauer Unwillen oder Abneigung gegen das Medikament auftreten und den Erfolg der Behandlung zunichte machen. Darüber hinaus ergibt sich aus der häufigen Einnahme von Wirkstoffmengen, welche den wirksamen Blutspiegel immer wieder deutlich übersteigen, die Gefahr von Nebenwirkungen, vor allem im Magendarmtrakt (EP 153 104).

Deshalb möchte man seit langem schon über eine Arzneiform verfügen, welche vom Wirkstoff eine höhere Dosis als etwa 300 oder 400 mg enthalten (EP 172 014) und den Inhalt über eine längere Zeitspanne freigeben würde. Auf diese Weise liessen sich die Häufigkeit der Einnahmen und die Anzahl Dosiseinheiten/Tag herabsetzen und gleichzeitig auch die bisher unumgänglichen Schwankungen des Blutspiegels zwischen einer sehr hohen und einer sehr tiefen Konzentration an Ibuprofen (fluctuating index) weitgehend ausmerzen.

Gemäss EP 172 014 können mit Hilfe des Natriumsalzes von vernetzter Carboxymethylcellulose Granulate und daraus Tabletten mit sehr hohem Ibuprofengehalt - z.B. 1000 mg - hergestellt werden. Diese Tabletten zeichnen sich jedoch durch eine recht hohe Auflösungsgeschwindigkeit in Wasser aus, so dass von 1000 mg Wirkstoff nach etwa 12 Minuten bereits 90% in Lösung vorliegen. Insofern lassen sich durch dieses Präparat die erwähnten Schwankungen des Blutspiegels zwischen hoher und tiefer Ibuprofenkonzentration nicht verringern.

Unterscheiden muss man zuerst bei den sogenannten Retard-Präparaten zwischen solchen mit verzögerter Freisetzung (delayed release) und solchen mit verlängerter Freisetzung (extended or sustained release) des Wirkstoffes, wie in Pharmacopeial Forum 14 (5), 4403-4405 (1988) festgehalten wird. Bei den ersteren soll der Wirkstoff erst im Darm aus dem Präparat abgegeben werden, wofür der pH-Unterschied zwischen dem Magen und dem Darm zunutze gemacht wird. Der Wirkstoff wird z.B. gemäss GB 2 132 887 mit einem bei pH 1 bis 6 unlöslichen Ueberzug umhüllt, der sich nach dem Magendurchgang im Darmsaft rasch auflöst. Bei den Präparaten mit verlängerter Wirkung hingegen soll der Wirkstoff von allem Anfang, d.h. bereits vom Zeitpunkt der Einnahme an seine Wirkung ausüben und diese über eine möglichst lange Zeitspanne fortsetzen.

An Vorschlägen in dieser Richtung hat es zwar nicht gefehlt. So beschreiben DE 2 908 794 und JP 81-30402 A feste Präparate zur verzögerten Freigabe von Ibuprofen und anderen physiologisch aktiven Substanzen; darin befindet sich der Wirkstoff in einer im Magensaft oder erst im Darmsaft löslichen Polymermatrix eingebettet. Die Herstellung erfolgt, indem man in einer wässrigen Dispersion des Wirkstoffes und eines oder mehrerer verglasbaren Monomeren, wie Ethylenglykol- oder Triethylenglykoldimethacrylat, unterhalb 0° C, vorzugsweise zwischen -20 und -80° C, durch Bestrahlung, zweckmässig mit $\gamma$-Strahlen die Polymerisation bewirkt. Die Präparate werden in Form von Mikrokügelchen oder harten Filmen erhalten.

Gemäss BE 903 540 werden für Wirksfoffe aller Art und jeglicher Wirkungsrichtung, u.a. Ibuprofen, sowie auch für Saccharin, Aspartam usw., Pulver mit verzögerter Freigabe hergestellt. Dazu werden aus einem Lösung oder Dispersion des Wirkstoffes in einem nicht toxischen Polymeren, z.B. Cellulosederivate, Polyacrylsäure- und Polymethacrylsäurederivate, Polyvinylverbindungen, Polysiloxane, Polyurethane usw., die Lösungs- oder Dispersionsmittel entfernt und dadurch Mikropartikeln erhalten.

Die erwähnten Verfahren scheinen allerdings in der Technik bisher keine Anwendung gefunden zu haben. Zurückzuführen ist dies zuerst auf den beträchtlichen apparativen Aufwand und die Schwierigkeiten, welche mit der Einleitung und Steuerung einer Polymerisation bei Temperaturen von -20 bis -80 °C oder mit dem Entfernen eines Lösungsmittels aus einer Lösung bis zu den letzten Spuren einhergehen. Vor allem aber ist es praktisch unmöglich, nach diesen Verfahren feste Präparate mit einem hohen Gehalt an Ibuprofen herzustellen.

Dennoch sind Retardpräparate erschienen, wie u.a. die Produkte Dolgit® retard oder Novogent® N (Rote Liste, Editio Cantor, Aulendorf/BRD 1984), in welchen das Ibuprofen in Kapseln zu 400 bzw. 300 mg vorliegt. Es werden davon üblicherweise 2 mal 2 Kapseln/Tag verabreicht; die tägliche Gesamtdosis kann bis zu 6 Kapseln betragen. Ebenfalls 300 mg Ibuprofen enthalten Hartgelatine-Kapseln mit verzögerter Freigabe des Wirkstoffes gemäss EP-Anmeldung 61 217; empfohlen wird die Einnahme von zwei Kapseln zweimal täglich, nach jeweils 12 Stunden. Die Herstellung erfolgt in einem Dragierkessel; über einen spherischen Kern aus Zucker und Stärke wird pulverförmiges Ibuprofen mittels eines Polyvinylpyrrolidons von niedriger Viskosität gebunden und die Spheroide werden anschliessend mit einem hochviskosen Polyvinylpyrrolidon überzogen und in Kapseln abgefüllt.

Bekanntlich haben die grössten Kapseln des Handels - Standardgrösse 0 oder 00 - ein Fassungsvermögen für Sphäroide oder Pellets, welches höchstens 350 mg Wirkstoff entspricht. Deshalb ist das oben erwähnte Behandlungsschema erforderlich, obschon es die eingangs gestellten Anforderungen keineswegs erfüllt. Zudem bedingt das Herstellungsverfahren einen erheblichen Arbeitsaufwand durch das mehrmalige zeitraubende Ueberziehen und Eindampfen des Lösungsmittels.

Diese Präparate erbringen zwar die geforderte Retardwirkung, sie erfüllen jedoch den Zweck insofern nicht, als die gegenüber den herkömmlichen Tabletten unveränderte Höhe der Dosiseinheit (300 oder 400 mg) die erwünschte Reduktion der Einnahmen nicht zulässt.

Andererseits sind Versuche zur Herstellung von Retard-Präparaten mit einem Wirkstoffgehalt von beispielsweise 600 bis 800 mg gescheitert. In der Tat hat Ibuprofen einen niedrigen Schmelzpunkt (75-77 °C), welcher bei Mischung mit den üblichen Exzipienten noch weiter herabgesetzt wird. Infolgedessen genügt der Druck der Tablettierungsphase, um den Wirkstoff teilweise zum Schmelzen zu bringen. Dadurch wird die feste Masse klebrig, was die Herstellung von Tabletten beträchtlich erschwert. Diese Umstände wirken sich besonders nachteilig bei der Herstellung von Retardpräparaten aus, welche öfters Fettmassen oder ähnliche niedrigschmelzende Hilfsstoffe enthalten, beispielsweise Lipide wie hydrierte Oele oder Paraffine gemäss JP 84-122 425 A.

Aus demselben Grund liesse sich die Lehre von GB 2 178 313 auf die Herstellung eines Retard-Präparates von Ibuprofen nicht übertragen, wird doch gemäss der Patentschrift das zunächst überzogene Granulat des Wirkstoffes während mindestens 5 Minuten auf einer Temperatur von 50 bis 120 °C, vorzugsweise während 15 Minuten bis einer oder zwei Stunden auf 60 bis 90 °C erhitzt. Ein Sintern und Zusammenbacken des Ibuprofen enthaltenden Materials wäre unvermeidlich und würde jede weitere Verarbeitung verunmöglichen.

Gemäss EP 153 104 und EP 52 075 schliesslich werden pharmazeutische Wirkstoffe in kristalliner oder granulierter Form mit einem homogenen Gemisch aus einem Polyacrylsäureester und einem wasserunlöslichen Cellulosederivat umhüllt. Dadurch wird ein körniges Granulat erhalten, aus welchem die Freisetzung des Wirkstoffes zwar verzögert verläuft, jedoch nach dem Verpressen zu Tabletten unverändert bleibt. Durch den erwähnten Filmüberzug wird also den ursprünglichen Kristallen oder Granulaten gute Komprimierbarkeit verleiht, ohne dass demgegenüber die Wirkstoffabgabe aus den Tabletten verändert wird. Die Umhüllung erfolgt dadurch, dass das homogene Gemisch aus Polyacrylsäureester und wasserunlöslichem Cellulosederivat im Wirbelschichtverfahren bei 40 bzw. 35 °C auf den Wirkstoff gesprüht oder mit diesem vermischt und extrudiert wird.

Entsprechende Versuche mit Ibuprofen sind jedoch fehlgeschlagen; es entstand jeweils eine derart klebrige und weiche, plastische Masse, dass sich deren Extrusion und nachfolgende Verarbeitung zu Mikrosphären praktisch nicht durchführen liess. Wie oben bereits erläutert wird, erklärt sich dieser Misserfolg durch den niedrigen Schmelzpunkt des Ibuprofens und dessen Verhalten in Mischung mit Exzipienten.

Trotz allen Versuchen war es also bisher nicht gelungen, von Ibuprofen eine Arzneiform zu schaffen, von welcher dank hohem Gehalt an Wirkstoff und verzögerter Freigabe im Organismus nur je 1 Einheit nur zweimal täglich verabreicht werden könnte.

Es ist nun ein pharmazeutisches Präparat in Form von Tabletten gefunden worden, welche Ibuprofen in einer überraschend hohen Dosis enthalten, ein für den Patienten dennoch annehmbares Ausmass (Volumen) aufweisen und den Wirkstoff im Organismus über eine längere Zeitspanne regelmässig freigeben, so dass die schon vor langer Zeit gestellten Anforderungen endlich erfüllt werden.

Die erfindungsgemässen Tabletten enthalten den Wirkstoff in Mikrosphären, zerfallen in wässrigem Medium rasch zu den Mikrosphären und haben folgende Zusammensetzung:

a) Ibuprofen in einer Menge von mindestens 600 mg und

b) ein Bindemittel oder ein Gemisch von Bindemitteln auf der Basis von Cellulose und wasserlöslichen Cellulosederivaten,

welche beide in homogener Mischung und in Form von Mikrosphären vorliegen, die ihrerseits mit einer Schicht

c) eines Acrylharzes von neutralem Charakter und mittlerem Molekulargewicht um 800'000, bestehend aus Copolymeren von Acrylsäure- und Methacrylsäureestern der Teilformel:

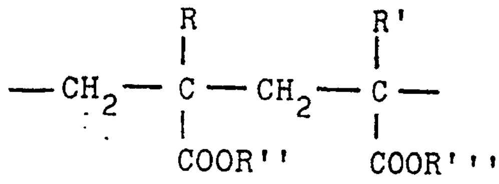

in welcher R und R' Wasserstoff oder Methyl und R" und R"' Methyl oder Ethyl bedeuten,

überzogen sind und die überzogenen Mikrosphären in homogener Mischung mit

d) einem Gemisch von Sprengmitteln, Gleitmitteln und Komprimierungshilfsstoffen, welches nur 10 bis 15 Gew.-% der überzogenen Mikrosphären ausmacht,

zu Tabletten zusammengepresst sind.

Gemäss einer bevorzugten Ausführungsform sind die Tabletten mit einer Lackschicht überzogen.

Hergestellt werden die erfindungsgemässen Tabletten, indem man das Ibuprofen und das erwähnte Bindemittel oder Gemisch von Bindemitteln zu einer homogenen Mischung miteinander vermischt und mit Wasser befeuchtet, die Mischung durch Extrusion zu Mikrosphären formt, die Mikrosphären trocknet, mit einer wässrigen Dispersion eines wie oben definierten Acrylharzes überzieht und trocknet, die derart überzogenen Mikrosphären unter Zusatz von 10 bis 15% Gew.-% eines Gemischs von Sprengmitteln, Gleitmitteln und Komprimierungshitfsstoffen, bezogen auf die überzogenen Mikrosphären, zu einer homogenen Mischung vermischt, die Mischung zu Tabletten presst und die erhaltenen Tabletten gewünschtenfalls mit einer Lackschicht überzieht.

Als Bindemittel eignen sich insbesondere Cellulose und wasserlösliche Cellulosederivate wie Methylcellulose, Carboxymethylcellulose (auch Celluloseglykolat genannt), Hydroxyethylcellulose, Hydroxypropylcellulose und Celluloseethansulfonsäure. Siehe auch dazu Ullmanns Encyklopädie der technischen Chemie, 4. Auflage, Band 9 Seiten 192-212, Verlag Chemie GmbH, Weinheim (BRD) 1975.

Unter den vielen Acrylharzen der oben angegebenen Beschaffenheit hat sich für die Zwecke der Erfindung ganz besonders jenes Äthyl(meth)acrylat-Polymerisat bewährt, das im Handel unter dem Markennamen Eudragit E 30 D (Röhm Pharma GmbH, Darmstadt, BRD) zu finden ist.

Als Sprengmittel kann man unter anderem ein Polyvinylpyrrolidon, Stärke, einen Stärkeether, Carboxymethylstärke (auch Stärkeglykolat genannt), ein Pektin und andere übliche Quellmittel wie Cellulose usw. verwenden.

Von Ibuprofen kann die erfindungsgemässe Tablette so hohe Mengen wie beispielsweise 600 bis 1200 mg oder sogar mehr enthalten. In der Tat hängt diese Menge nicht etwa von dem Volumen ab, das man den Patienten in Form einer Tablette zu schlucken maximal zumuten kann, und ebenso wenig von dem Fassungsvermögen einer im Handel befindlichen Kapsel, sondern allein von der Form und dem Ausmass des Stempels der Tablettiermaschine.

## Beispiele

Hergestellt werden Tabletten der folgenden Zusammensetzung :

| | | | Beispiel 1 | Beispiel 2 | Beispiel 3 |
|---|---|---|---|---|---|
| A | Ibuprofen | | 800,0 mg | 800,0 mg | 600,0 mg |
| A | mikrokristalline Cellulose Avicel® PH 102 | (1) | 130,0 " | 133,3 " | 100,0 " |
| A | mikrokristalline Cellulose mit 8 % Carboxymethylcellulose Avicel® RC 581 | (1) | 130,0 " | 133,3 " | 100,0 " |
| A | Hydroxypropylcellulose Pharmcoat® 603 | (2) | 40,0 " | 40,0 " | 30,0 " |
| | Acrylharz Eudragit® E 30 D | (3) | 53,0 " | 53,3 " | 40,0 " |

Beispiele (Fortsetzung)

| | | | Beispiel 1 | Beispiel 2 | Beispiel 3 |
|---|---|---|---|---|---|
| B | hochdisperses Siliciumdioxid Aerosil® 200 | (4) | 15,0 mg | 13,0 mg | 10,0 mg |
| | Magnesiumstearat | | 12,0 " | 12,0 " | 9,0 " |
| | Talk | | 30,0 " | 60,0 " | 45,0 " |
| | quervernetzte, unlösliche Homopolymere von N-Vinyl-2-pyrrolidon Crospovidone NF Polyplasdone® XL 10 | (5) | 50,0 " | — | — |
| | Natriumsalz einer niedrig substituierten Carboxymethylstärke US-Pharmakopöe XXI Explotab®, Primojel® | (6) | 10,0 " | 60,0 " | 45,0 " |
| | | | 1270 mg | 1305 mg | 979 mg |

(1) Hersteller : FMC Corporation, Chicago (IL, USA)
(2) Hersteller : Shin-Etsu Chemical Co., Cellulose Div., Tokyo (Japan)
(3) Hersteller : Röhm Pharma GmbH, Darmstadt (BRD)
(4) Hersteller : Degussa AG, Frankfurt a/Main (BRD)
(5) Hersteller : GAF Corporation, Wayne (NJ, USA)
(6) Hersteller : AVEBE International Marketing and Sales, Foxhol (Die Niederlande)

Die mit A bezeichneten Stoffe werden miteinander, unter Befeuchtung mit Wasser oder einer wässrigen Lösung der Bindemittel gemischt und die feuchte Mischung wird durch einen Extruder und einen Spheronizer (Abrundungsmaschine) zu Mikrosphären geformt, welche anschliessend in einem Luftstrom bei ca. 45°C getrocknet werden. Die getrockneten Mikrosphären werden mit einer wässrigen Suspension des angegebenen Acrylharzes im Wirbelschichtverfahren bei einer Temperatur von etwa 30 °C überzogen. Hierauf

werden die überzogenen Mikrosphären mit den unter B angeführten Stoffen in einem Mischapparat vermischt und die erhaltene Mischung wird zu Tabletten gepresst. Vorzugsweise werden die Tabletten anschliessend in einem Dragierkessel mit einer Lackschicht überzogen.

Von den gemäss den Beispielen 1, 2 und 3 hergestellten Tabletten ist die Auflösungsgeschwindigkeit in Wasser nach der folgenden Methode bestimmt worden :

| | |
|---|---|
| - Vorschrift: | United States Pharmacopoeia XXI (1985), Seite 1243 |
| - Apparatur: | Apparat 1, mit 150 U/Min. rotierendes Körbchen |
| - Medium : | wässriger Phosphatpuffer, pH 6,5 |
| - Volumen : | 900 ml |
| - Volumen der Probe : | 1 ml |
| - Zeitpunkt der Probenahme : | nach 30, 60, 120, 180, 240, 300, 360, 420 Minuten |
| - Messung : | spektroskopisch bei 221 nm UV |

Die Ergebnisse zeigen, dass im Lauf der Zeit Ibuprofen aus den Tabletten in folgendem Mengenverhältnis (Durchschnittswerte) freigesetzt wird:

| | Beispiel 1 | Beispiele 2 und 3 |
|---|---|---|
| nach 1 Stunde | 21,6 % | 27,6 % |
| " 2 Stunden | 37,8 % | 42,8 % |
| " 4 Stunden | 61,9 % | 64,4 % |
| " 7 Stunden | 80,5 % | 76,8 % |
| " 10 Stunden | 99,7 % | 94,0 % |

Mit anderen Worten wird in wässrigem Medium der Wirkstoff innert etwa 10 Stunden aus den erfindungsgemässen Tabletten praktisch quantitativ freigesetzt. Aus dem zeitlichen Verlauf der Freisetzung wird zudem ersichtlich, dass die Tabletten rasch zerfallen und dass die Freisetzung regelmässig fortschreitet. Aus diesen Gründen kann sich im Organismus schon kurze Zeit nach der Einnahme ein wirksamer und anhaltender Blutspiegel einstellen, wie in den klinischen Untersuchungen festgestellt wurde.

Um die Wirksamkeit des Präparates zu prüfen, wurde seine biologische Verfügbarkeit (bioavailability) in zwei getrennten in vivo-Prüfungen bestimmt und mit jener von anderen galenischen Formulierungen des Ibuprofens verglichen. Die erwähnten in vivo-Prüfungen wurden im August, September und Oktober bzw. November und Dezember 1984 von IPHAR, Institut für Klinische Pharmakologie GmbH, Höhenkirchen (BRD), an fünf bzw. acht freiwilligen, gesunden, männlichen Probanden durchgeführt.

Zum Vergleich gelangten in der ersten in vivo-Prüfung die folgenden Retard-Präparate:

(A) Novogent® N, Kapseln zu 300 mg
Hersteller: Temmler-Werke, Vereinigte Chemische Fabriken, Marburg (BRD)

(B) Dolgit® retard, Kapseln zu 400 mg
Hersteller: Dolorgiet Arzneimittelfabrik Peter Doll KG, Bonn (BRD)

(C) MP 031, Lacktabletten zu 600 mg gemäss Beispiel 3 (erfindungsgemäss)

und ein Präparat ohne verzögerte Wirkung, nämlich :

(D) Brufen® , Dragées zu 400 mg
Hersteller: Adolf Klinge u. Co., München (BRD)

Jeder Proband erhielt jeweils im Abstand von einer Woche eine orale Einzeldosis von 400 mg Dolgit retard (B) bzw. Brufen (D) und 600 mg Novogent N (2 Kapseln zu 300 mg, A) bzw. eine Lacktablette MP 031 zu 600 mg (C).

In der zweiten in vivo-Prüfung wurden die folgenden Retard-Präparate:

- MP 031, Lacktabletten zu 600 mg gemäss Beispiel 3 (erfindungsgemäss)
- MP 031·, Lacktabletten zu 800 mg gemäss Beispiel 1 (erfindungsgemäss)
- Fenbid® retard, Kapseln zu 300 mg
  Hersteller: Smith, Kline & French Laboratories Ltd., Welwyn Garden City (Hertfordshire, England)

und das bereits erwähnte Präparat ohne verzögerte Wirkung

- Brufen® , Dragées zu 400 mg
  Hersteller: Adolf Klinge u. Co., München (BRD)

miteinander verglichen. Jeder der acht Probanden erhielt jeweils im Abstand von einer Woche eine orale

Einzeldosis von 600 mg MP 031, 800 mg MP 031·, 600 mg Fenbid retard (2 Kapseln zu 300 mg) bzw. 800 mg Brufen (2 Dragées zu 400 mg).

Jedem Probanden wurden gemäss einem bestimmten Zeitplan Blutproben entnommen und daraus das Plasma gewonnen und auf die Konzentration an Ibuprofen analysiert. Die Bestimmung erfolgte in Anlehnung an die Methode von S.F. Lockwood und J.C. Wagner [Journal of Chromatography 232 (1982), 335-343] durch ein Hochdruck-Flüssigchromatographie-Verfahren.

Aus den zu verschiedenen Zeitpunkten gemessenen Konzentrationen an Ibuprofen im Plasma wurden die in den folgenden Tabellen zusammengefassten Parameter berechnet:

- die maximale Konzentration, $C_{max}$
- die Zeitspanne bis zur maximalen Konzentration, $t_{max}$
- die Fläche unter der Kurve der Konzentrationen, AUC
- die mittlere Verweildauer, MRT, und
- die Eliminationshalbwertzeitszeit, $t_{1/2}$

Tabelle 1

| | Parameter | Proband 1 | 2 | 3 | 4 | 5 | X̄ | SD |
|---|---|---|---|---|---|---|---|---|
| A | Cmax | 5.30 | 15.17 | 13.36 | 9.73 | 13.95 | 11.95 | 4.49 |
| | tmax | 2.5 | 6.0 | 2.5 | 3.0 | 2.5 | 3.4 | 1.8 |
| | AUC | 56.38 | 134.23 | 140.36 | 93.15 | 127.70 | 114.67 | 39.20 |
| | MRT | 8.82 | 8.16 | 10.60 | 9.65 | 9.83 | 9.35 | 1.08 |
| | $t_{1/2}$ | 8.36 | 5.77 | 6.27 | 5.11 | 5.35 | 6.44 | 1.34 |
| B | Cmax | 10.86 | 16.14 | 17.07 | 10.42 | 7.06 | 12.78 | 4.69 |
| | tmax | 4.0 | 4.0 | 2.5 | 2.5 | 12.0 | 5.6 | 4.3 |
| | AUC | 66.00 | 108.35 | 133.49 | 65.81 | 115.04 | 105.72 | 28.53 |
| | MRT | 6.07 | 5.61 | 6.94 | 5.37 | 14.14 | 8.19 | 4.00 |
| | $t_{1/2}$ | 2.21 | 2.30 | 3.65 | 2.96 | | 2.72 | 0.81 |
| C | Cmax | 3.71 | 23.11 | 25.92 | - | 12.25 | 16.25 | 10.23 |
| | tmax | 4.0 | 4.0 | 3.0 | - | 4.0 | 3.8 | 0.5 |
| | AUC | 27.05 | 142.24 | 198.84 | - | 157.46 | 131.40 | 73.56 |
| | MRT | 6.79 | 6.31 | 7.53 | - | 10.89 | 7.88 | 2.07 |
| | $t_{1/2}$ | 3.52 | 2.45 | 4.48 | - | 3.56 | 3.50 | 0.83 |
| D | Cmax | 4.34 | 37.05 | 37.89 | 27.33 | 12.07 | 22.84 | 17.19 |
| | tmax | 1.5 | 0.8 | 1.5 | 1.5 | 8.0 | 2.9 | 3.4 |
| | AUC | 13.54 | 92.47 | 140.07 | 76.47 | 131.22 | 94.32 | 57.69 |
| | MRT | 2.69 | 2.43 | 4.04 | 3.21 | 9.11 | 4.57 | 3.11 |
| | $t_{1/2}$ | 1.65 | 1.57 | 2.11 | 1.64 | 3.99 | 2.33 | 1.13 |

Pharmakokinetische Parameter von Ibuprofen ermittelt in 5 gesunden Probanden nach oraler Gabe von Novogent N (A, 600 mg), Dolgit retard (B, 400 mg), MP 031 (C, 600 mg) bzw. Brufen (D, 400 mg)

$C_{max}$ = maximale Plasmakonzentration ($\mu g\ ml^{-1}$)
$t_{max}$ = Zeit bis zur maximalen Plasmakonzentration (h)
AUC = Fläche unter der Plasmaspiegelkurve ($\mu g\ ml^{-1}.h$)
MRT = mittlere Verweildauer (h)
$t_{1/2}$ = Eliminationshalbwertszeit (h)

X̄ = Mittelwert
SD = Standardabweichung

Tabelle 2

| | Parameter | Proband 1 | 2 | 3 | 4 | 5 | 6 | 7 | 8 | $\bar{X}$ | SD |
|---|---|---|---|---|---|---|---|---|---|---|---|
| MP 031 | Cmax | 14.41 | 16.08 | 27.21 | 27.25 | 17.40 | 7.33 | 19.14 | 15.63 | 18.06 | 6.63 |
| | tmax | 2.5 | 4.0 | 5.0 | 2.5 | 6.0 | 6.0 | 3.0 | 6.0 | 4.4 | 1.6 |
| | AUC | 160.62 | 207.48 | 239.78 | 175.46 | 170.49 | 121.29 | 127.56 | 162.66 | 170.67 | 38.94 |
| | MRT | 10.69 | 9.71 | 8.59 | 7.03 | 8.42 | 10.01 | 6.42 | 9.30 | 8.77 | 1.47 |
| MP 031• | Cmax | 11.60 | 14.51 | 27.60 | 25.20 | 17.02 | 15.18 | 21.36 | 19.54 | 19.00 | 5.50 |
| | tmax | 2.5 | 7.0 | 5.0 | 6.0 | 4.0 | 6.0 | 6.0 | 3.0 | 4.9 | 1.6 |
| | AUC | 196.71 | 225.73 | 271.31 | 245.60 | 201.03 | 179.65 | 189.37 | 195.70 | 213.14 | 31.63 |
| | MRT | 12.06 | 10.93 | 8.85 | 8.84 | 9.28 | 10.69 | 8.44 | 9.11 | 9.77 | 1.29 |
| FENBID | Cmax | 14.78 | 13.36 | 22.11 | 21.72 | 13.39 | 25.69 | 14.85 | 17.52 | 17.93 | 4.68 |
| | tmax | 5.0 | 4.0 | 3.0 | 5.0 | 3.0 | 2.5 | 2.5 | 3.0 | 3.5 | 1.0 |
| | AUC | 169.79 | 175.44 | 212.87 | 159.97 | 137.36 | 154.15 | 124.21 | 152.35 | 160.77 | 26.76 |
| | MRT | 10.92 | 9.55 | 8.68 | 6.01 | 7.34 | 6.28 | 7.14 | 7.22 | 7.89 | 1.69 |
| BRUFEN | Cmax | 49.01 | 59.29 | 90.39 | 54.57 | 56.37 | 49.11 | 27.12 | 55.36 | 55.15 | 17.41 |
| | tmax | 1.0 | 1.5 | 2.0 | 2.5 | 0.5 | 2.5 | 1.0 | 1.5 | 1.6 | 0.7 |
| | AUC | 318.04 | 285.44 | 377.91 | 214.23 | 249.14 | 178.93 | 179.21 | 262.60 | 258.19 | 68.88 |
| | MRT | 6.00 | 6.02 | 5.92 | 7.97 | 3.77 | 7.61 | 6.16 | 4.86 | 6.04 | 1.35 |
| | $t_{1/2}$ | 4.1 | 2.5 | 2.8 | 2.9 | 1.6 | 2.5 | 4.1 | 1.8 | 2.8 | 0.9 |

**Pharmakokinetische Parameter von Ibuprofen ermittelt in 8 gesunden Probanden nach oraler Gabe von MP 031 (600 mg), MP 031•(800 mg), Fenbid retard (600 mg) bzw. Brufen (800 mg).**

Cmax = maximale Plasmakonzentration ($\mu g\ ml^{-1}$)
tmax = Zeit bis zur maximalen Plasmakonzentration (h)
AUC = Fläche unter der Plasmaspiegelkurve ($\mu g\ ml^{-1}.h$)
MRT = mittlere Verweildauer (h)
$t_{1/2}$ = Eliminationshalbwertszeit (h)

$\bar{X}$ = Mittelwert

SD = Standardabweichung

Zwischen den Zeitkurven der Plasmakonzentration der einzelnen galenischen Verabreichungsformen traten deutliche Unterschiede auf. Bei Brufen (D) waren die Werte der maximalen Plasmakonzentration $C_{max}$ am höchsten und die Zeitspanne bis zur Erreichung der maximalen Konzentration $t_{max}$ am kürzesten, und die Werte der mittleren Verweildauer MRT sowie die Halbwertszeit $t_{1/2}$ lagen am niedrigsten, wie von einer nicht retardierten Formulierung auch zu erwarten war.

Die berechneten Werte der Flächen unter den Konzentrationskurven AUC stellen das Ausmass der Bioverfügbarkeit der einzelnen Formulierungen dar. Aus Tabelle 1 geht hervor, dass die Bioverfügbarkeit für das erfindungsgemässe Präparat (C) mit dem AUC-Wert 131,40 am höchsten ist, was ohne Zweifel auf das Zusammenwirken des höheren Gehalts an Wirkstoff und dessen verzögerten Freigabe zurückgeht. Gemäss Tabelle 2 weisen mit 170,67 bzw. 213,14 die erfindungsgemässen Präparate MP 031 und MP 031· unter

den retardierten Formulierungen ebenfalls den höchsten AUC-Wert, d.h. die höchste Bioverfügbarkeit, auf.

Der entscheidende Vorteil liegt aber darin, dass durch die Erfindung ein Präparat geschaffen wird, welches in einer Einheitsdosis von längerer Wirkungsdauer eine mindestens 2- bis 3-fach höhere Wirkstoffmenge enthält als es bisher möglich war. Es war jedoch durchaus nicht zu erwarten, dass ein Zusatz von nur 10 bis 15 % an äusserer Phase (in den Beispielen 1, 2 und 3 die unter B angeführten Stoffe gesamthaft) zu den Mikrosphären die Herstellung einer Tablette ermöglichen würde, welche bei einem Gehalt an Ibuprofen von 600 mg oder mehr ein nicht zu grosses bzw. noch annehmbares (d.h. schluckbares) Ausmass aufweisen und in wässrigem Medium so rasch wie jede herkömmliche (d.h. durch Zusammenpressen eines Granulats oder eines Pulvers entstandene) Tablette zerfallen würde.

Die im Magen durch den Zerfall freigewordenen Mikrosphären "fliessen" sozusagen fortlaufend in den Darm hinüber, weitgehend unabhängig von der zeitweise erfolgenden Entleerung des Magens, und entfalten ihre Retardwirkung während der ganzen Transitdauer. Dank der hervorragenden Wirksamkeit, die sich in der biologischen Verfügbarkeit zeigt, wird also der Medizin ein Präparat zur Verfügung gestellt, welches eine erfolgreiche Behandlung der oben erwähnten Indikationen bei minimalen Anforderungen an den Patienten (Anzahl der täglichen Einnahmen und Anzahl der Dosiseinheiten pro Einnahme oder pro Tag) ermöglicht.

**Patentansprüche**

**Patentansprüche für folgende Vertragsstaaten: BE, DE, FR, GB, IT,**
LU, NL, SE

**1.** Pharmazeutisches Präparat zur verzögerten Freigabe von Ibuprofen im Organismus, dadurch gekennzeichnet, dass es als Tabletten vorliegt, welche den Wirkstoff in Mikrosphären enthalten, in wässrigem Medium rasch zu den Mikrosphären zerfallen und folgende Zusammensetzung haben:

a) Ibuprofen in einer Menge von mindestens 600 mg und

b) ein Bindemittel oder ein Bindemittelgemisch auf der Basis von Cellulose und wasserlöslichen Cellulosederivaten,

welche zusammen in homogener Mischung und in Form von Mikrosphären vorliegen, die ihrerseits mit einer Schicht

c) eines Acrylharzes von neutralem Charakter und mittlerem Molekulargewicht um 800'000, bestehend aus Copolymeren von Acrylsäure- und Methacrylsäureestern der Teilformel:

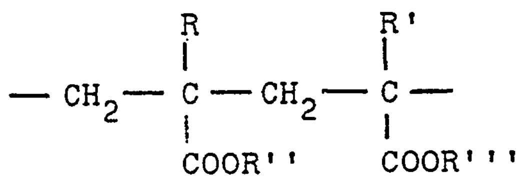

in welcher R und R' Wasserstoff oder Methyl und R'' und R''' Methyl oder Ethyl bedeuten,

überzogen sind, und die überzogenen Mikrosphären in homogener Mischung mit

d) einem Gemisch von Sprengmitteln, Gleitmitteln und Komprimierungshilfsstoffen, welches nur 10 bis 15 Gew.-% der überzogenen Mikrosphären ausmacht,

zu Tabletten zusammengepresst sind.

**2.** Präparat nach Anspruch 1, dadurch gekennzeichnet, dass die Tabletten mit einer Lackschicht überzogen sind.

**3.** Präparat nach Anspruch 1 oder 2, dadurch gekennzeichnet, dass es 600 bis 1200 mg Ibuprofen pro Tablette enthält.

**4.** Präparat nach Anspruch 1 oder 2, dadurch gekennzeichnet, dass das Bindemittel mikrokristalline Cellulose und ein wasserlösliches Cellulosederivat enthält.

**5.** Präparat nach Anspruch 1 oder 2, dadurch gekennzeichnet, dass das Acrylharz aus Äthyl(meth)acrylat-Polymerisat (Eudragit® E 30 D) besteht.

**6.** Präparat nach Anspruch 1 oder 2, dadurch gekennzeichnet, dass das Sprengmittel Cellulose, Stärke,

ein Stärkeether, ein Pektin oder ein ähnlich wirkendes übliches Quellmittel ist.

7. Verfahren zur Herstellung des pharmazeutischen Präparates nach einem der Ansprüche 1 bis 6, dadurch gekennzeichnet, dass man das Ibuprofen in einer Menge, welche pro Tablette mindestens 600 mg entspricht, und das Bindemittel oder das Bindemittelgemisch auf der Basis von Cellulose und wasserlöslichen Cellulosederivaten zu einer homogenen Mischung miteinander vermischt und mit Wasser befeuchtet, die Mischung durch Extrusion zu Mikrosphären formt, die Mikrosphären trocknet, mit einer wässrigen Dispersion eines Acrylharzes von neutralem Charakter und mittlerem Molekulargewicht um 800'000, bestehend aus Copolymeren von Acrylsäure- und Methacrylsäureestern der Teilformel:

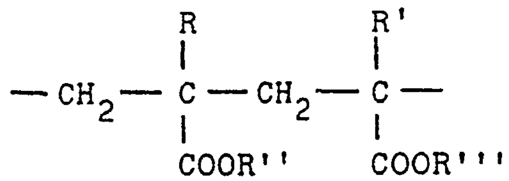

in welcher R und R' Wasserstoff oder Mehtyl und R'' und R''' Methyl oder Ethyl bedeuten, überzieht und trocknet, die überzogenen Mikrosphären unter Zusatz von 10 bis 15 Gew.-% eines Gemischs von Sprengmitteln, Gleitmitteln und Komprimierungshilfsstoffen, bezogen auf die überzogenen Mikrosphären, zu einer homogenen Mischung vermischt und die Mischung zu Tabletten presst.

8. Verfahren nach Anspruch 7, dadurch gekennzeichnet, dass man die erhaltenen Tabletten ausserdem mit einer Lackschicht überzieht.

9. Verfahren nach Anspruch 7 oder 8, dadurch gekennzeichnet, dass Ibuprofen in einer Menge von 600 bis 1200 mg pro Tablette eingesetzt wird.

10. Verfahren nach einem der Ansprüche 7 bis 9, dadurch gekennzeichnet, dass als Acrylharz Äthyl(meth)-acrylat-Polymerisat (Eudragit® E 30 D) eingesetzt wird.

**Patentansprüche für folgende Vertragsstaaten: AT, ES, GR**

1. Verfahren zur Herstellung eines Retard-Präparates von Ibuprofen, dadurch gekennzeichnet, dass man Ibuprofen in einer Menge, welche mindestens 600 mg pro Tablette entspricht, und ein Bindemittel oder ein Bindemittelgemisch auf der Basis von Cellulose und wasserlöslichen Cellulosederivaten zu einer homogenen Mischung miteinander vermischt und mit Wasser befeuchtet, die Mischung durch Extrusion zu Mikrosphären formt, die Mikrosphären trocknet, mit einer wässrigen Dispersion eines Acrylharzes von neutralem Charakter und mittlerem Molekulargewicht um 800'000, bestehend aus Copolymeren von Acrylsäure- und Methacrylsäureestern der Teilformel:

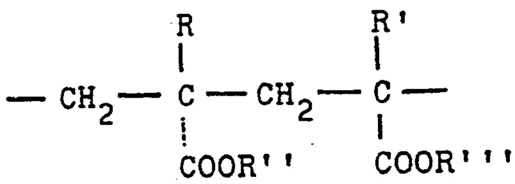

in welcher R und R' Wasserstoff oder Methyl und R'' und R''' Methyl oder Ethyl bedeuten, überzieht und trocknet, die überzogenen Mikrosphären unter Zusatz von 10 bis 15 % Gew.-% eines Gemischs von Sprengmitteln, Gleitmitteln und Komprimierungshilfsstoffen, bezogen auf die überzogenen Mikrosphären,zu einer homogenen Mischung vermischt und die Mischung zu Tabletten presst, welche den

Wirkstoff in Mikrosphären enthalten und in wässrigem Medium rasch zu den Mikrosphären zerfallen.

2. Verfahren nach Anspruch 1, dadurch gekennzeichnet, dass man die erhaltenen Tabletten ausserdem mit einer Lackschicht überzieht.

3. Verfahren nach Anspruch 1 oder 2, dadurch gekennzeichnet, dass Ibuprofen in einer Menge von 600 bis 1200 mg pro Tablette eingesetzt wird.

4. Verfahren nach Anspruch 1 oder 2, dadurch gekennzeichnet, dass das Bindemittel mikrokristalline Cellulose und ein wasserlösliches Cellulosederivat enthält.

5. Verfahren nach Anspruch 1 oder 2, dadurch gekennzeichnet, dass als Acrylharz Äthyl(meth)acrylat-Polymerisat (Eudragit® E 30 D) verwendet wird.

6. Verfahren nach Anspruch 1 oder 2, dadurch gekennzeichnet, dass als Sprengmittel Cellulose, Stärke, ein Stärkeether, ein Pektin oder ein ähnlich wirkendes übliches Quellmittel verwendet wird.

**Claims**

**Claims for the following Contracting States: BE, DE, FR, GB,** IT, LU, NL, SE

1. A pharmaceutical preparation for the sustained release of ibuprofen in the body, characterized in that it is in the form of tablets which contain the active compound in microspheres, disintegrate fast in an aqueous medium, and have the following composition:
   a) ibuprofen in an amount of at least 600 mg and
   b) a binder, or a mixture of binders, based on cellulose and water-soluble cellulose derivatives,

   all of which are in the form of a homogeneous mixture and in the form of microspheres which, in turn, are coated with a layer of
   c) an acrylic resin of neutral character and average molecular weight of around 800,000, composed of copolymers of acrylic and methacrylic esters of the partial formula:

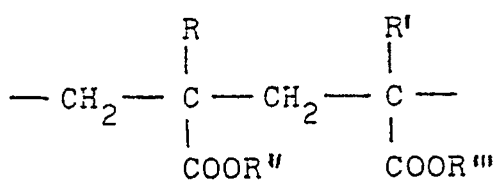

   in which R and R' denote hydrogen or methyl, and R'' and R''' denote methyl or ethyl,

   and the coated microspheres are homogeneously mixed with
   d) a mixture of disintegrants, lubricants and auxiliary materials for compressing, which constitutes only 10 to 15 wt.% of the coated microspheres, and are compressed to form tablets.

2. The preparation as claimed in claim 1, wherein the tablets are coated with a lacquer layer.

3. The preparation as claimed in claim 1 or 2, which contains 600 to 1200 mg of ibuprofen per tablet.

4. The preparation as claimed in claim 1 or 2, wherein the binder contains microcrystalline cellulose and a water-soluble cellulose derivative.

5. The preparation as claimed in claim 1 or 2, wherein the acrylic resin is composed of ethyl (meth)-acrylate polymerisate (Eudragit® E 30 D).

6. The preparation as claimed in claim 1 or 2, wherein the disintegrant is cellulose, starch, a starch ether, a pectin or a customary swelling agent of similar action.

7. A process for producing the pharmaceutical preparation as claimed in any one of claims 1 to 6, which

comprises mixing together the ibuprofen in an amount which corresponds to at least 600 mg per tablet and the binder, or the mixture of binders, based on cellulose and water-soluble cellulose drivatives to give a homogeneous mixture and moistening with water, shaping the mixture into microspheres by extrusion, drying the microspheres, coating with an aqueous dispersion of an acrylic resin of neutral character and average molecular wight of around 800,000, composed of copolymers of acrylic and methacrylic esters of the partial formula:

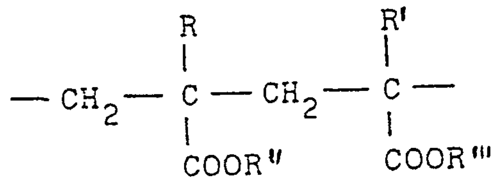

in which R and R' denote hydrogen or methyl, and R'' and R''' denote methyl or ethyl, and drying, mixing the coated microspheres with an additive of from 10 to 15 wt.%, based on the coated microspheres, of a mixture of disintegrants, lubricants and auxiliary materials for compressing to give a homogeneous mixture, and compressing the mixture to form tablets.

**8.** The process as claimed in claim 7, wherein the resulting tablets are additionally coated with a lacquer layer.

**9.** The process as claimed in claim 7 or 8, wherein ibuprofen is used in an amount of 600 to 1200 mg per tablet.

**10.** The process as claimed in any one of claims 7 to 9, wherein ethyl (meth)acrylate polymerisate (Eudragit® E 30 D) is used as acrylic resin.

**Claims for the following Contracting States: AT, ES, GR**

**1.** A process for producing a sustained release ibuprofen preparation, which comprises mixing together ibuprofen in an amount which corresponds to at least 600 mg per tablet and a binder, or a mixture of binders, based on cellulose and water-soluble cellulose derivatives to give a homogeneous mixture and moistening with water, shaping the mixture into microspheres by extrusion, drying the microspheres, coating with an aqueous dispersion of an acrylic resin of neutral character and average molecular weight of around 800,000, composed of copolymers of acrylic and methacrylic esters of the partial formula:

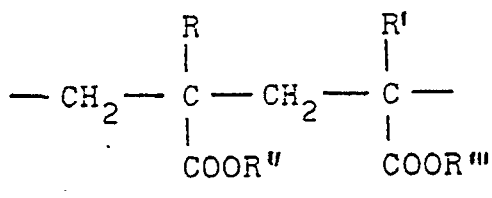

in which R and R' denote hydrogen or methyl, and R'' and R''' denote methyl or ethyl, and drying, mixing the coated microspheres with an additive of from 10 to 15 wt.%, based on the coated microspheres, of a mixture of disintegrants, lubricants and auxiliary materials for compressing to give a homogeneous mixture, and compressing the mixture to form tablets which contain the active compound within microspheres and in an aqueous medium fast disintegrate to said microspheres.

**2.** The process as claimed in claim 1, wherein the resulting tablets are additionally coated with a lacquer layer.

**3.** The process as claimed in claim 1 or 2, wherein ibuprofen is used in an amount of 600 to 1200 mg per tablet.

4. The process as claimed in claim 1 or 2, wherein the binder contains microcrystalline cellulose and a water-soluble cellulose derivative.

5. The process as claimed in claim 1 or 2, wherein ethyl (meth)acrylate polymerisate (Eudragit® E 30 D) is used as acrylic resin.

6. The process as claimed in claim 1 or 2, wherein cellulose, starch, a starch ether, a pectin or a customary swelling agent of similar action is used as disintegrant.

**Revendications**
**Revendications pour les Etats contractants suivants: BE, DE, FR,**
GB, IT, LU, NL, SE

1. Composition pharmaceutique pour la libération Revendications pour les Etats contractants suivants: BE, DE, FR, GB, IT, LU, NL, SE retardée d'ibuprofène dans l'organisme, caractérisée en ce qu'elle se présente sous forme de comprimés qui contiennent le principe actif dans des microsphères, se désagrègent rapidement en milieu aqueux en les microsphères et ont la composition suivante:
   a) ibuprofène en une quantité d'au moins 600 mg et
   b) un agent de liaison ou un mélange d'agents de liaison à base de cellulose ou de dérivés de cellulose solubles dans l'eau,
qui sont ensemble en mélange homogène et sous forme de microsphères, lesquelles sont revêtues d'une couche
   c) d'une résine acrylique de nature neutre et d'un poids moléculaire moyen d'environ 800'000, consistant en copolymères d'esters de l'acide acrylique et de l'acide méthacrylique répondant à la formule partielle:

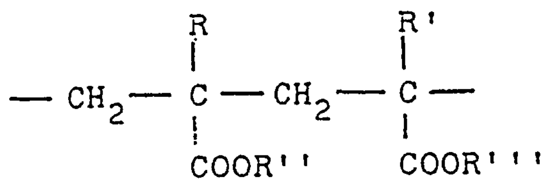

   dans laquelle R et R' représentent l'hydrogène ou le méthyle et R'' et R''' représentent le méthyle ou l'éthyle,
et les microsphères revêtues, en mélange homogène avec
   d) un mélange de désintégrants, d'agents d'écoulement et d'auxiliaires de compression, qui ne constitue que 10 à 15% en poids des microsphères revêtues,
sont assemblées en forme de comprimés.

2. Composition selon la revendication 1, caractérisée en ce que les comprimés sont revêtus d'une couche de laque.

3. Composition selon la revendication 1 ou 2, caractérisée en ce qu'elle contient de 600 à 1200 mg d'ibuprofène par comprimé.

4. Composition selon la revendication 1 ou 2, caractérisée en ce que l'agent de liaison contient de la cellulose microcristalline ou un dérivé de cellulose soluble dans l'eau.

5. Composition selon la revendication 1 ou 2, caractérisée en ce que la résine acrylique consiste en un polymère de (méth)acrylate d'éthyle (Eudragit® E 30 D).

6. Composition selon la revendication 1 ou 2, caractérisée en ce que le désintégrant est de la cellulose, de l'amidon, un éther d'amidon, une pectine ou un agent gonflant usuel de même effet.

7. Procédé de préparation de la composition pharmaceutique selon l'une des revendications 1 à 6,

caractérisé en ce que l'on mélange l'un avec l'autre, en un mélange homogène, l'ibuprofène en une quantité qui corresponde à au moins 600 mg par comprimé et l'agent de liaison ou le mélange d'agents de liaison à base de cellulose et de dérivés de cellulose solubles dans l'eau, et qu'on humecte avec de l'eau, qu'on façonne le mélange en microsphères par extrusion, qu'on sèche les microsphères, qu'on revêt d'une dispersion aqueuse d'une résine acrylique de nature neutre et de poids moléculaire moyen d'environ 800'000, consistant en copolymères d'esters de l'acide acrylique et de l'acide méthacrylique répondant à la formule partielle:

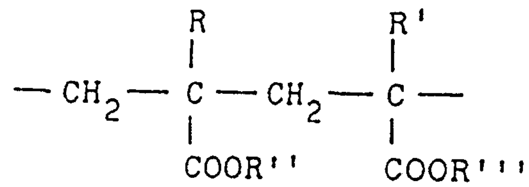

dans laquelle R et R' représentent l'hydrogène ou le méthyle et R" et R'" représentent le méthyle ou l'éthyle, et qu'on sèche, qu'on mélange les microsphères revêtues avec adjonction de 10 à 15% en poids, par rapport aux microsphères revêtues, d'un mélange de désintégrants, d'agents d'écoulement et d'auxiliaires de compression, pour former un mélange homogène, et qu'on comprime le mélange en comprimés.

**8.** Procédé selon la revendication 7, caractérisé en ce que, en outre, on revêt les comprimés obtenus d'une couche de laque.

**9.** Procédé selon la revendication 7 ou 8, caractérisé en ce que l'on met en jeu l'ibuprofène en une quantité de 600 à 1200 mg par comprimé.

**10.** Procédé selon l'une des revendications 7 à 9, caractérisé en ce que l'on met en jeu comme résine acrylique un polymère de (méth)acrylate d'éthyle (Eudragit® E 30 D).

**Revendications pour les Etats contractants: AT, ES, GR**

**1.** Procédé de préparation d'un médicament retard d'ibuprofène, caractérisé en ce que l'on mélange l'un avec l'autre, en un mélange homogène, l'ibuprofène en une quantité qui corresponde à au moins 600 mg par comprimé et un agent de liaison ou un mélange d'agents de liaison à base de cellulose et de dérivés de cellulose solubles dans l'eau, et qu'on humecte à l'eau, qu'on façonne le mélange en microsphères par extrusion, qu'on sèche les microsphères, qu'on revête d'une dispersion aqueuse d'une résine acrylique de nature neutre et d'un poids moléculaire moyen d'environ 800'000, consistant en copolymères d'esters de l'acide acrylique et de l'acide méthacrylique répondant à la formule partielle:

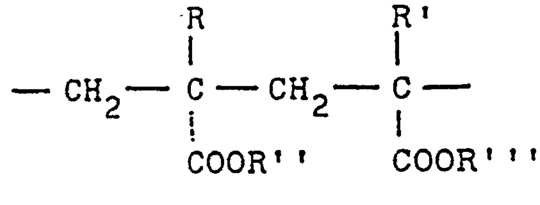

dans laquelle R et R' représentent l'hydrogène ou le méthyle et R" et R'" représentent le méthyle ou l'éthyle, et qu'on sèche, qu'on comprime les microsphères revêtues, avec adjonction de 10 à 15% en poids, par rapport aux microsphères revêtues, d'un mélange de désintégrants, d'agents d'écoulement et d'auxiliaires de compression, pour former un mélange homogène et que l'on comprime le mélange en comprimés qui contiennent le principe actif en des microsphères et se désagrègent rapidement en

microsphères en milieu aqueux.

2. Procédé selon la revendication 1, caractérisé en ce que, en outre, on revêt les comprimés obtenus d'une couche de laque.

3. Procédé selon la revendication 1 ou 2, caractérisé en ce que l'on met en jeu l'ibuprofène en une quantité de 600 à 1200 mg par comprimé.

4. Procédé selon la revendication 1 ou 2, caractérisé en ce que l'agent de liaison contient de la cellulose microcristalline ou un dérivé de cellulose soluble dans l'eau.

5. Procédé selon la revendication 1 ou 2, caractérisé en ce que l'on utilise comme résine acrylique un polymère de (méth)acrylate d'éthyle (Eudragit® E 30 D).

6. Procédé selon la revendication 1 ou 2, caractérisé en ce que l'on utilise comme désintégrant de la cellulose, de l'amidon, un éther d'amidon, une pectine ou un agent gonflant usuel d'effet semblable.